# EUROPEAN PATENT APPLICATION

(11) **EP 2 426 191 A1**
(43) Date of publication of application: **07.03.2012**
(21) Application number: 10008054.8
(22) Date of filing: 02.08.2010
(51) Int. Cl.: C11C 3/12, C07C 5/10, C07C 5/11, C07C 29/141, C07C 229/76, C07D 213/79, C07D 215/50, C07D 215/52, C07D 471/04, C07D 487/22

(54) **Process for the homogenous hydrogenation of (poly)unsaturated hydrocarbons**

(71) Applicant: Cognis IP Management GmbH, 49589 Düsseldorf (DE); National and Kapodistrian University of Athens, 10561 Athens (GR)
(72) Inventor: Papadogianakis, Georgios, 13121 Ilion, Athens (GR); Bouriazos, Achilleas, 18542 Palaia Kokkínía, Peíreas (GR)
(74) Representative: Fabry, Bernd

(57) **Abstract**

Suggested is a new process for the selective hydrogenation of (poly)unsaturated hydrocarbons, in particular polyunsaturated fatty acid alkyl esters into the respective saturated and/or monounsaturated species, of aldehydes into respective alcohols and of aromatic compounds into respective saturated cyclic compounds using homogenous catalysts in a biphasic aqueous/organic solvent system, which is characterised in that said catalysts represent complexes of main-group I-V metals with water-soluble amine ligands.

## Description

### Field of the invention

The present invention is related to the area of homogenous catalysis and refers to the hydrogenation of (poly)unsaturated hydrocarbons, in particular esters, glycerides, aldehydes and aromatic compounds employing new water-soluble and non-toxic complexes of main-group I-V metals and amine ligands.

### Background of the invention

The field of transition metal catalyzed hydrogenation reactions in heterogeneous and homogeneous systems has found numerous laboratory scale applications and is a very important unit process in the oil refining-, bulk/fine chemicals- and pharmaceutical-industry.

In sharp contrast, there are only a few reports in the field of main-group metal catalyzed homogeneous hydrogenation reactions and all of them are performed in conventional organic media. In 2008, Spielmann et al. [Angew. Chem. lnt. Ed. 47 p 9434-9438 (2008**)]** described the homogeneous hydrogenation of activated alkenes such as 1,1,-diphenylethylene and of myrcene catalyzed by calcium, strontium and potassium modified by bidentate amine ligands in conventional organic media such as benzene and THF. Emphasis was placed on the sensitivity of this reaction to the polarity of the solvent which has a strong influence on the σ-bond metathesis step and that polar conditions accelerate the whole hydrogenation reaction. However, the scope of this hydrogenation is limited to starting materials with conjugated double bonds. In 2010, Barrett et al. [Proc. R. Soc. A 466 p 927-963 (2010**)]** reviewed the field of homogeneous hydrogenation, hydroamination, hydrosilylation and hydrophosphination reactions of alkenes, alkynes, dienes, carbodiimides, isocyanates and ketones catalyzed by complexes of early main-group metals such as calcium, strontium and barium in conventional organic media. In 2009, Westerhausen [Z. Anorg. Allg. Chem. 635 p 13-32 (2009**)**] reported on recent developments in organocalcium chemistry including calcium-catalyzed polymerization, hydroamination, hydrophosphination, hydrosilylation and Tishchenco-type reactions in conventional organic media. In 2010, Zeng et al. [Inorg. Chem. 49 p 3361-3369 (2010**)**] studied mechanistic aspects of the Ca/amine-catalyzed homogeneous hydrogenation of conjugated alkenes in conventional organic media. Finally, it is worthwhile to be mentioned that recently, in 2010, *Power* reviewed ***[***Nature 463, p 171-177 (2010**)]** the field of the late main-group elements which resembles that of transition metal complexes.

In this context, the selective hydrogenation of polyunsaturated fatty acid alkyl esters, for example based on sunflower oil, to obtain high oleic acid alkyl esters - useful for biodiesel applications - represents an area of serious commercial interest. Although various process have been reported using homogenous or heterogeneous catalysts, the results still leave room for improvement, either with respect to selectivity, turnover numbers, conditions, and - of course - availability, costs and recovery of the respective catalysts.

In general, C=C bonds are easier hydrogenated by homogeneous catalytic systems compared with carbonyl groups which are more difficult to be reduced with the same technique. Therefore, there is an enormous interest in the development of efficient homogeneous catalysts for the hydrogenation of aliphatic-, aromatic- and arylaliphatic-aldehydes into respective alcohols and especially for the selective hydrogenation of a,β-unsaturated aldehydes which are valuable intermediates in the fragrance- and flavor-chemistry.

The hydrogenation of benzene to cyclohexane is one of the most important industrially practiced aromatic compounds hydrogenation reaction. However, despite its apparent simplicity the hydrogenation reaction of benzene has evolved through many variations and has given rise to many different processes. The successful industrial hydrogenation of benzene suitable for petrochemical cyclohexane production requires the resolution of three major critical problems: (i) the reaction is strongly exothermic with a ðH^{o}_{f (473K}) of - 214.2 kJ.mol⁻¹, (ii) the cyclohexane must be pure, and (iii) the low stability of e.g. nickel catalysts which require extremely pure benzene feedstocks with less than 1 ppm sulfur in order that the catalysts remain effective in the liquid-phase under mild conditions. The strongly exothermic hydrogenation reaction requires careful control of temperature, pressure and residence time in order to achieve quantitative conversion of benzene at very high cyclohexane selectivity

it is the object of the present invention to develop a new process for the hydrogenation of unsaturated species, in particular esters, glycerides, aldehydes and aromatic compounds in general and in particular for the selective transfer of polyunsaturated into saturated or monounsaturated species, of aldehydes into alcohols and of aromatic compounds into saturated cyclic compounds employing highly active, non-toxic, water-soluble main-group metal catalytic complexes in aqueous/organic two-phase systems.

### Detailed description of the invention

The present invention refers to a process for the selective hydrogenation of (poly)unsaturated hydrocarbons, in particular polyunsaturated fatty acid alkyl esters into the respective saturated and/or monounsaturated species, of aldehydes into respective alcohols and of aromatic compounds into respective saturated cyclic compounds using homogenous water-soluble catalysts in a biphasic aqueous/organic solvent system, which is characterised in that said catalysts represent complexes of main group I-V metals with water-soluble amine ligands.

Surprisingly it has been observed that employing the new water-soluble catalysts in associated with a great number of benefits:
o The novel class of highly active water-soluble catalytic complexes is based on main-group metals such as calcium which is very economic and one of the most common elements (2.79% of earth consists of calcium) and is in accordance with the concept *"Cheap Metals for Noble Tasks" ,* which means that the process is more economic compares to others using expensive noble metal complexes;
o The superiority of the new generation of industrially applied calcium catalysts is also based on its non-toxicity in the case of leaching of calcium in inorganic, biochemical and biological systems. It is possible to renounce from separating off the catalysts after hydrogenation, even in case the products are dedicated for nutrition purposes;
o The new catalysts include water-soluble ligands such as EDTA which are very economic and also representing very stable systems;
o The catalysts allow the use of water as a solvent, what renders the process cheap, easy and sustainable; due to the large heat capacity of water it is an excellent medium to perform exothermic reactions more safe and selective which is especially important in industrial large scale exothermic catalytic processes such as the hydrogenation processes;
o The water-soluble Ca/EDTA complexes according to the present invention are highly active (TOF > 63,000 h⁻¹), stable and robust catalytic systems, can be easily recovered - if necessary - by a simple phase separation and showed in recycling experiments that the catalytic activity remained high in consecutive runs;
o Using water-soluble Ca/TSTPP and Ca/EDTA catalytic complexes very low contents of trans-C18:1 esters of only 2.0 and 4.2 mol%, respectively, were obtained with reasonable catalytic activities of TOF > 4,400 h⁻¹ under mild reaction conditions at 60 °C and 50 bar of hydrogen pressure within 10 min of reaction time and at calcium concentrations of only 20 and 40 ppm in water, which is also interesting in the field of edible oil biphasic hydrogenation producing lower amounts of undesirable trans-fats.

Since the hydrogenation takes place in the unsaturated hydrocarbon chain the nature of the functional group of the respective molecule is not a critical issue. One may employ tri-, di-, or mono-glycerides, acids, alkylesters, amides, aldehydes, aromatic compounds or even pure hydrocarbons. Nevertheless, Figure 1 illustrates the overall process starting with a polyunsaturated fatty acid methyl ester obtained from sunflower oil ("MESO")

The term "regiomers" refers to all regio-isomers obtained from hydrogenation and/or positional isomerization (along the carbon chain) reactions and all other geometric isomers formed via *cis*/*trans* isomerization reactions.

Preferably, the (poly)unsaturated hydrocarbons contain at least two and at most five double or even triple bonds. In particular, the process encompasses the hydrogenation of hydrocarbons having 6 to 54, preferably 16 to 36 and more preferably 18 to 26 carbon atoms. Examples of suitable starting materials for the selective hydrogenation cover in particular fatty acid Cₗ-C₂₂ alkyl- or polyol esters, preferably fatty acid methyl or ethyl esters or glycerides, having 6 to 54, preferably 16 to 36 and more preferably 18 to 26 carbon atoms and 2 to 5 double bonds in the acyl moiety. In particular preferred is the selective hydrogenation of methyl and/or ethyl and/or glycerol esters of linolenic acid, linoleic acid and so-called omega-3 fatty acids, like eicosapentaenoic acid (EPA) docosahexaenoic acid (DHA) and their mixtures. Suitable sources for the starting material are also plant oils, like for example sunflower oil, rapeseed oil, olive oil, linseed oil, fish oil and the like, which are rich in polyunsaturated fatty acid, or their transesterification products withC₁-C₂₂, preferably C₁-C₄ alcohols, like methanol, ethanol or butanol. All these starting materials may contain mono unsaturated or even saturated species in lower amounts. 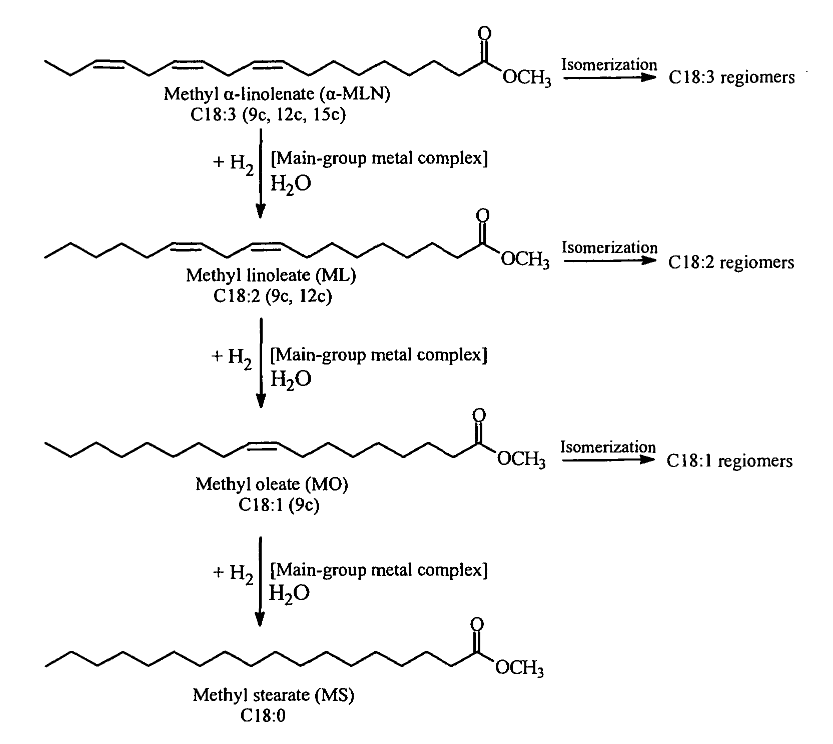 Figure 1: Hydrogenation process

The selection of aldehydes starting materials is not critical, since the process according to the present invention is suitable to convert all of aldehydes i.e. aliphatic-, aromatic-, arylaliphatic, unsaturated aliphatic or α,β,-unsaturated aldehydes into respective alcohols. The process according to the present invention in the example of hydrogenation of benzaldehyde into benzylalcohol follows the reaction which is shown below:

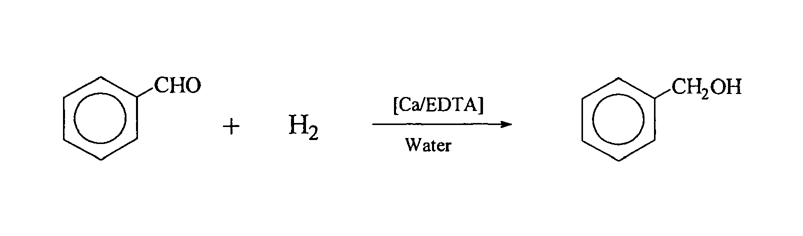

The selection of the cyclic unsaturated, usually aromatic mono- or polycyclic starting material is not critical, since the process according to the present invention is suitable to convert all of these unsaturated starting materials into their saturated homologues. Typically, suitable starting materials follow the following formulas 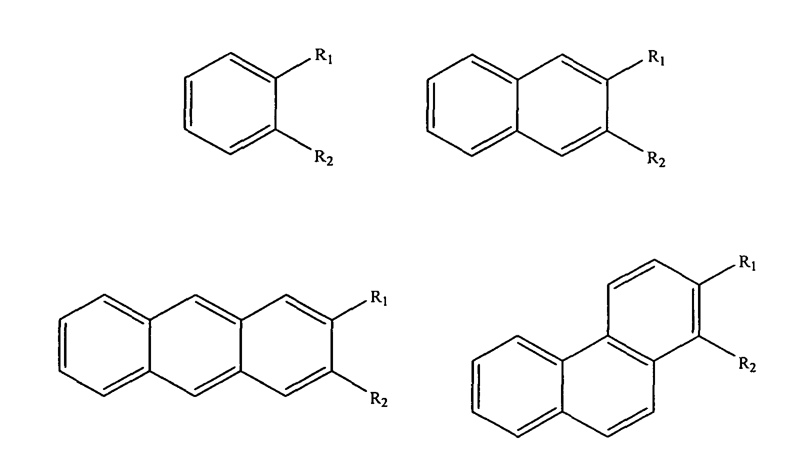 in which R¹ and R² independently from each other represent hydrogen or an alkyl, ester, alkoxy, aldehyde, keto, acid, amide, nitril, amino, nitro, halide, and/or hydroxy group. Also included are those geometric isomers of the cyclic compounds described above, that means those isomers which do not stand in 1,2 position, but in each other position to the first substituent, since the distribution of the substituents at the ring system does not have an influence on the hydrogenation process. Also encompassed are those cyclic compounds which do not show an aromatic behaviour, since they only comprise one or two double bonds. Even cyclic compounds like cyclopentadien or dicyclopentadien can be converted in the respective saturated compounds by means of the present process. The preferred cyclic starting materials, however, are benzene, toluene and dimer fatty acids or their esters, which are for example obtainable from the dimerisation of oleic acid. The process according to the present invention in the example of hydrogenation of benzene into cyclohexane follows the reaction which is shown below:

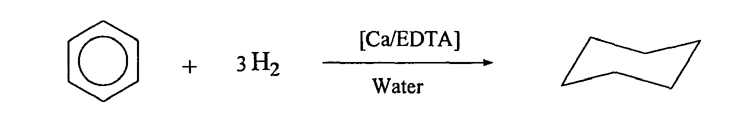

### Main-group Metal I to V Catalysts

The main-group metal catalysts represent complexes with calcium, magnesium, strontium, barium, lithium, sodium, potassium, rubidium, cesium, aluminium, gallium, indium, thallium, germanium, tin, lead, antimony or bismuth as their central atom. Most preferred catalysts represent complexes of calcium. The main group I-V metals are typically present in the form of their salts, for example as chlorides or acetates.

### Ligands

The following figure explains possible structure of water-soluble amine ligands to the main group metal catalysts, without limiting them to these examples:

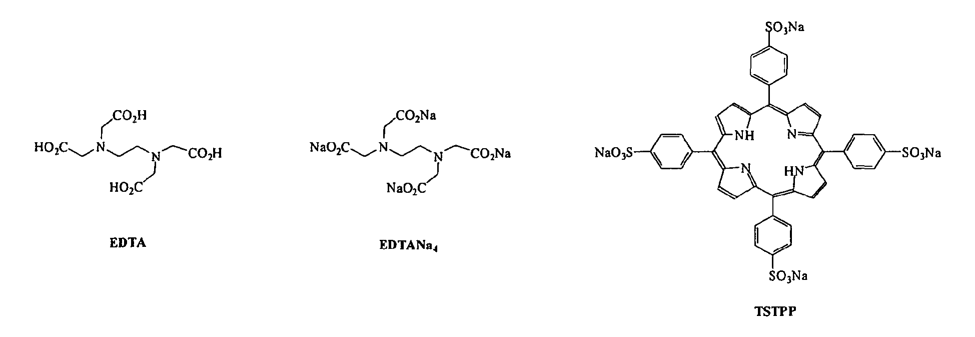

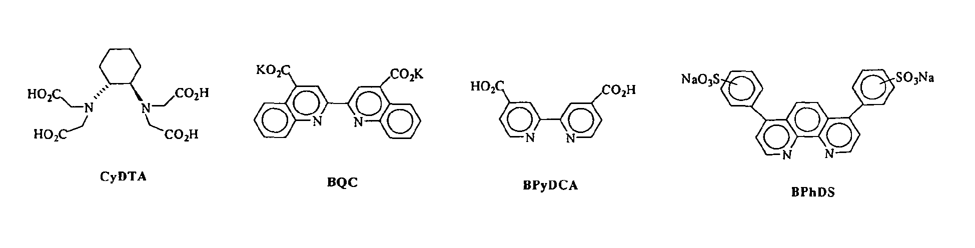

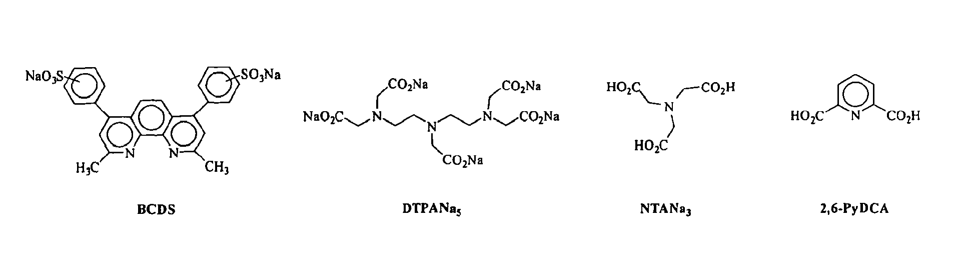

Useful examples encompass the following structures: ethylenediaminetetraacetic acid (EDTA), ethylenediaminetetraacetic acid tetrasodium salt (EDTANa₄), meso-tetra(sulfonatophenyl)porphine tetrasodium salt (TSTPP), trans-1,2-diaminocyclohexane-N,N,N',N'-tetraacetic acid (CyDTA), 2,2'-biquinoline-4,4'dicarboxylic acid dipotassium salt (BQC), 2,2-bipyridine-4,4-dicarboxylic acid (BPyDCA), bathophenanthrolinedisulfonic acid disodium salt (BPhDS), bathocuproinedisulfonic acid disodium salt (BCDS), diethylenetriaminepentaacetic acid pentasodium salt (DTPANa₅), nitrilotriacetic acid trisodium salt (NTANa₃) and 2,6-pyridinedicarboxylic acid (2,6-PyDCA). Said ligands can be combined with each of the main group I-V central metal atoms, preferred, however, are complexes between said ligands and calcium. In particular preferred are complexes of calcium and ethylenediaminetetraacetic acid (EDTA) or ethylenediaminetetraacetic acid tetrasodium salt (EDTANa₄).

### Co-catalysts

It has been found that the presence of cationic, zwitterionic (amphoteric), nonionic and anionic surfactants, ionic liquids (IL) and/or phase transfer catalysts (PTC) improves activity and selectivity of the catalyst and facilitates the separation of the complexes after the hydrogenation has taken place.

### Cationic and amphoteric surfactants

Typical examples for suitable cationic surfactants are the so-called tetraalkylammonium salts, which preferably follow general formula (I): 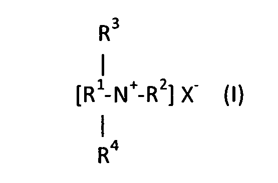 in which R¹, R², R³ and R⁴ independently from each other represent linear or branched, saturated or unsaturated aliphatic or hydroxyaliphatic radicals having 1 to 22 carbon atoms and X stands for chloride or bromide. Preferably at least two of these aliphatic radicals represent methyl groups, while at least one radical is a longer alkyl group having 8 to 18 carbon atoms. Typical examples are octyltrimethylammonium chloride (OTAC), dodecyltrimethylammonium chloride (DTAC), tetradecyltrimethylammonium chloride, cetyltrimethylammonium chloride (CTAC), cetylpyridinium chloride, octadecyltrimethylammonium chloride, distearyldimethylammonium chloride (DSDMAC). Other cationic surfactants of tetraalkylammonium salts include: tetrapentylammonium chloride, tetrahexylammonium chloride, tetradecylammonium chloride.

In the alternative, also cationic surfactants of the so-called esterquat type are useful, which can be derived from diethanol methylamine (DMA) and especially triethanol amine (TMA). The latter are preferably following general formula (II) 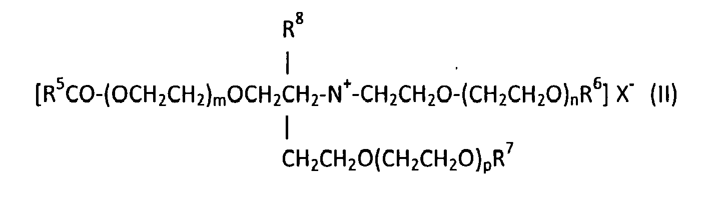 in which R⁵CO represents an acyl radical having 6 to 22, preferably 12 to 18 carbon atoms, R⁶ and R⁷ independently stand for hydrogen or R⁵CO, R⁸ means an alkyl radical having 1 to 4 carbon atoms or a (CH₂CH₂O)_{q}H-group, m, n and p represent in total 0 or an integer of 1 to 12, q represents numbers from 1 to 12 and X means halogenide, alkylsulphate or alkylphosphate. Typical and preferred examples are dicocoylmethylethoxymonium chloride and distearylmethylethoxymonium chloride which are sold under the trademark Dehyquart L80 or AU56 respectively.

Other suitable amphoteric surfactants among others are: 3-(N,N-dodecyldimethylammonium) propanesulphonate, 3-(N,N-tetradecyldimethylammonium) propanesulphonate, 3-(N,N-cetyldimethylammonium) propanesulphonate, 3-(N,N-octadecyldimethylammonium) propanesulphonate, N,N-dimethy-loctylamino-N-oxide and N,N-dimethyl-decylamino-N-oxide.

### Non-ionic surfactants

Also non-ionic surfactants can be added as co-catalyst, including for example:
o products of the addition of 2 to 30 mol ethylene oxide and/or 0 to 5 mol propylene oxide onto linear C₈-₂₂ fatty alcohols, onto C₁₂₋₂₂ fatty acids and onto alkyl phenols containing 8 to 15 carbon atoms in the alkyl group;
o C_{12/18} fatty acid monoesters and diesters of addition products of 1 to 30 mol ethylene oxide onto glycerol;
o alk(en)yl oligoglycosides;
o glycerol mono- and diesters and sorbitan mono- and diesters of saturated and unsaturated fatty acids containing 6 to 22 carbon atoms and ethylene oxide addition products thereof;
o addition products of 15 to 60 mol ethylene oxide onto castor oil and/or hydrogenated castor oil;
o polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
o addition products of 2 to 15 mol ethylene oxide onto castor oil and/or hydrogenated castor oil;
o partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
o mono-, di and trialkyl phosphates and mono-, di- and/or tri-PEG-alkyl phosphates and salts thereof;
o wool wax alcohols;
o polysiloxane/polyalkyl polyether copolymers and corresponding derivatives;
o mixed esters of pentaerythritol, fatty acids, citric acid and fatty alcohol and/or mixed esters of C₆-₂₂ fatty acids, methyl glucose and polyols, preferably glycerol or polyglycerol,
o polyalkylene glycols and
o glycerol carbonate.

The addition products of ethylene oxide and/or propylene oxide onto fatty alcohols, fatty acids, alkylphenols, alk(en)yl oligoglycosides, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### Alk(en)yl oligoglycosides

Alkyl or alkenyl oligoglycosides may be derived from aldoses or ketoses containing 5 or 6 carbon atoms, preferably glucose. Accordingly, the preferred alkyl and/or alkenyl oligoglycosides are alkyl or alkenyl oligoglucosides. These materials are also known generically as "alkyl polyglycosides" (APG). The alk(en)yl oligoglycosides according to the invention correspond to formula (III) :

R⁹O[G]_{P} (III)

wherein R⁹ is an alkyl or alkenyl radical having from 6 to 22 carbon atoms, G is a sugar unit having 5 or 6 carbon atoms and p is a number from 1 to 10. The index p in general formula (III) indicates the degree of oligomerisation (DP degree), i.e. the distribution of mono- and oligoglycosides, and is a number of 1 to 10. Whereas p in a given compound must always be an integer and, above all, may assume a value of 1 to 6, the value p for a certain alkyl oligoglycoside is an analytically determined calculated quantity which is mostly a broken number. Alk(en)yl oligoglycosides having an average degree of oligomerisation p of 1.1 to 3.0 are preferably used. Alk(en)yl oligoglycosides having a degree of oligomerisation below 1.7 and, more particularly, between 1.2 and 1.4 are preferred from the applicational point of view. The alkyl or alkenyl radical R⁹ may be derived from primary alcohols containing 4 to 22 and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred.

### Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### Polyglycerol esters

Typical examples of suitable polyglycerol esters are Polyglyceryl-2 Dipolyhydroxystearate (Dehymuls^{•} PGPH), Polyglycerin-3-Diisostearate (Lameform^{•} TGI), Polyglyceryl-4 Isostearate (Isolan Gi 34), Polyglyceryl-3 Oleate, Diisostearoyl Polyglyceryl-3 Diisostearate (Isolan^{®} PDI), Polyglyceryl-3 Methylglucose Distearate (Tego Care 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

In particular preferred examples for suitable nonionic surfactants of Brij-type are among others the following systems: 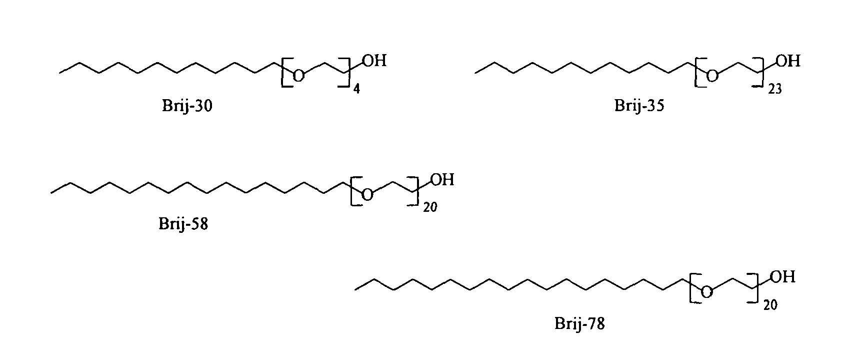 and of Triton X - type such as: 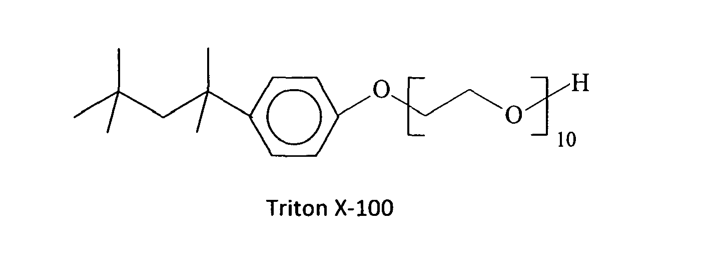 as well as Triton X-114, Triton X-305 and Triton X-405.

### Anionic surfactants

Anionic surfactants suitable as co-catalysts in this biphasic catalytic hydrogenation reaction are among others: sodium dodecylsulphate (SDS): 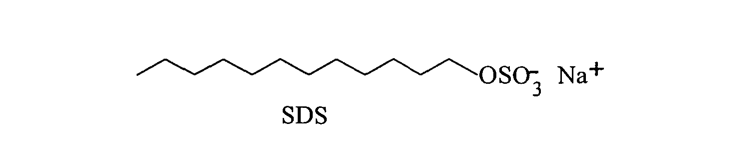 dodecylbenzonesulphonate (DBS), sodium 1-heptanesulphonate, sodium 1-octanesulphonate, sodium 1-nonanesulphonate, sodium 1-decanesulphonate and salts of linear alkylbenzenesulphonate (LABS).

### Phase transfer catalysts

Suitable phase transfer catalysts also useful as co-catalysts are summarised in J.Am.Chem.Soc., 93, p195f, (1971). Typical examples are tetraalkylammonium salts with short alkyl groups, benzyltrialkylammonium salts, tetraalkylphosphonium salts, benzyltrialkylphosphonium salts and their mixtures. In preferred embodiments of the present invention tetra-n-butylammonium, tri-n-butylmethylammonium, benzyltriethylammonium, tetra-n-butylphosphonium, tri-n-butylmethylphosphonium, benzyltriethylphosphonium in form of their chlorides, bromides or hydrogensulphates are used.

### Hydrogenation process

In the first step the catalyst is prepared, for example by simply dissolving the water-soluble main group I-V metal salt (e.g. Ca(OAC)₂) and the water-soluble ligand (e.g. the EDTA) in demineralised water which is not necessary to be deaerated. The aqueous catalyst system is mixed with the starting material, e.g. a polyunsaturated fatty acid alkyl ester or glyceride to result in a two-phase system formed by the aqueous catalyst solution and the polyunsaturated fatty acid alkyl ester or glyceride. The ratio between the volume of the aqueous and the organic phase is typically about 5:1 to about 1:5 and preferably about 1:1. The mixture comprising the ester or glyceride, the catalyst and optionally the organic solvent is transferred into a stirred autoclave and after a number of pressurising-depressurising cycles with hydrogen in order to remove air. Then the reactor is heated up to 50 to 130, preferably 70 to 80 °C at kept there for a reaction time of 5 to 240, preferably 10 to 30 minutes. During this time the pressure rises up to 5 to 100 bar. Once the reaction has been completed the mixtures is cooled to room temperature and depressurised. The upper organic layer comprising the hydrogenation product is separated from the lower aqueous layer and subjected to purification, e.g. drying and/or distillation. The aqueous layer containing the catalyst is recycled.

### Industrial application

An additional embodiment of the present invention is directed to complexes of main group I-V metals and water-soluble amine ligands, more particular ligands selected from the group consisting of ethylenediaminetetraacetic acid (EDTA), ethylenediaminetetraacetic acid tetrasodium salt (EDTANa₄), meso-tetra(sulfonatophenyl)porphine tetrasodium salt (TSTPP), *trans*-1,2-diaminocyclohexane-N,N,N',N'-tetraacetic acid (CyDTA), 2,2'-biquinoline-4,4'di-carboxylic acid dipotassium salt (BQC), 2,2-bipyridine-4,4-dicarboxylic acid (BPyDCA), bathophenanthrolinedisulfonic acid disodium salt (BPhDS), bathocuproinedisulfonic acid disodium salt (BCDS), diethylenetriaminepentaacetic acid pentasodium salt (DTPANa₅), nitrilotriacetic acid trisodium salt (NTANa₃) and 2,6-pyridinedicarboxylic acid (2,6-PyDCA).

Finally, the invention also encompasses the use of said water-soluble complexes as homogenous catalysts for the hydrogenation of (poly)unsaturated hydrocarbons, aldehydes and aromatic compounds, in particular for the selective hydrogenation of polyunsaturated fatty acid esters into saturated and/or monounsaturated fatty acid esters, of aldehydes into alcohols and of aromatic compounds into saturated cyclic compounds.

### Examples

### Examples 1 to 6

### Effect of the early main-group metal catalyst precursors modified by EDTANa_{4.}H₂O

A commercially available sunflower oil methyl ester (MESO) was subjected to hydrogenation using various homogenous catalysts in aqueous/organic two phase systems under the following conditions:
- ₀ T = 120 °C
- ⁰ P_{H2} = 50 bar
- a t = 10 min
- 0.01 mmol of early main-group metal catalyst precursor
- ₀ 3.98 mg (0.01 mmol) EDTANa₄·H₂O (EDTANa₄/M molar ratio = 1)
- ₀ 13.89 g (60 mmol of C=C units) of MESO
- ₀ molar ratio of C=_{C} units/Ca = 6 000
- 10 ml of demineralized water
- pH= 4.08 - 6.27

For the blank run the autoclave was thoroughly cleaned and than for 62 times the biphasic hydrogenation of MESO in 10 ml of water was carried out in the presence of various alkaline earth metal catalyst precursors modified by various water-soluble amine ligands mainly by EDTA at 120°C under 30 bar hydrogen partial pressure within 10 min and followed by numerous series of treatment of the autoclave at elevated temperatures (120°C) and pressures (50 bar of H₂) within 1 hour each time in the presence of TPPTS/water and PPh₃/MESO. After these treatments the blank run was carried out at T = 120 °C, P_{H2} = 50 bar within 10 min with addition of 10 ml of demineralized water and 13.89 g (60 mmol of C=_{C} units) of MESO which results a two-phase system with a volume ratio of aqueous/organic phase = 10/15.4 in the absence of any catalytic system in order to find out whether memory effects of the autoclave regarding transition metals are still operative. Subsequently, the addition of MESO to the aqueous catalyst solution results a two-phase system with a volume ratio of aqueous/organic phase = 10/15.4; stirring rate = 620 rpm.

The following Table 1 shows the effect of the early main-group metal catalyst precursors modified by EDTANa₄·H₂O. The turnover frequency (TOF) is defined as mole of hydrogenated C=C units in the C18:3, C18:2 and C18:1 compounds in both the starting material MESO mixture and all other regiomers formed during the course of the reaction per mole of main-group metal per hour.

**Table 1**

| Hydrogenation of sunflower oil methyl ester-composition in mol% | | | | | | | | |
|---|---|---|---|---|---|---|---|---|
| **Ex** | **Catalyst precursor** | **C18:3** | **C18:2** | **C18:1** | *Cis* **C18:1** | ***Trans* C18:1** | **C18:0** | **TOF [h ⁻¹]** |
| - | MESO | 1.3 | 59.0 | 36.5 | 36.5 | - | 3.2 | - |
| 0 | Blank run | 0.0 | 57.5 | 37.9 | 36.0 | 1.9 | z 4.6 | - |
| 1 | Mg(OAc)₂*4H₂O/ EDTANa₄*H₂O | 0.0 | 32.3 | 54.2 | 38.1 | 16.1 | 13.5 | 10100 |
| 2 | Ca(OAc)₂*H₂O/ EDTANa₄*H₂O | 0.0 | 20.3 | 56.7 | 36.0 | 20.7 | 23.0 | 14400 |
| 3 | Sr(OAc)₂*H₂O/ ED-TANa₄* H₂O | 0.0 | 24.5 | 55.0 | 41.1 | 13.9 | 20.5 | 12900 |
| 4 | Ba(OAc)_{2/} EDTANa₄*H₂O | 0.0 | 20.8 | 56.3 | 37.1 | 19.2 | 22.9 | 14300 |
| 5 | MgCl₆*6H₂O EDTANa₄*H₂O | 0.0 | 28.6 | 55.4 | 39.1 | 16.3 | 16.0 | 11500 |
| 6 | CaCl₂*2H₂O/ EDTANa₄*H₂O | 0.0 | 25.1 | 55.2 | 39.4 | 15.8 | 19.7 | 12700 |

### Examples 7 to 18

### Effect of water-soluble ligand modifiers on the calcium-catalyzed hydrogenation

A commercially available sunflower oil methyl ester (MESO) was subjected to hydrogenation using various homogenous catalysts in aqueous/organic two phase systems under the following conditions:
- T=120°C
- P_{H2} = 50 bar
- t =10 min
- 1.76 mg (0.01 mmol) Ca(OAC)₂·H₂O
- 0.01 mmol of the ligand (Ligand /Ca molar ratio = 1)
- 13.89 g (60 mmol of C=C units) of MESO; molar ratio of C=C units/Ca = 6 000; 10 ml of demineralized water
- [Ca] = 40 ppm
- pH= 3.91- 5.56

Addition of MESO to the aqueous catalyst solution results a two-phase system with a volume ratio of aqueous/organic phase = 10/15.4; stirring rate = 620 rpm. The following Table 2 shows the effect of water-soluble amine ligand modifiers on the calcium-catalyzed hydrogenation of MESO in aqueous/organic two-phase systems

**Table 2 Hydrogenation of sunflower oil methyl ester-composition in mol%**

| **Ex** | **Catalyst precursor** | **C18:3** | **C18:2** | **C18:1** | ***Cis* C18:1** | ***Trans* C18:1** | **C18:0** | **TOF [h⁻¹]** |
|---|---|---|---|---|---|---|---|---|
| - | MESO | 1.3 | 59.0 | 36.5 | 36.5 | - | 3.2 | - |
| 7 | Ca(OAc)₂*H₂O | 0.0 | 20.6 | 57.0 | 34.6 | 22.4 | 22.4 | 14300 |
| 8 | Ca(OAc)₂*H₂O/ DTPANaS | 0.0 | 17.1 | 56.3 | 38.0 | 18.3 | 26.6 | 15600 |
| 9 | Ca(OAc)₂*H₂O/ EDTA | 0.0 | 18.1 | 53.6 | 32.0 | 21.6 | 28.3 | 15200 |
| 10 | Ca(OAc)₂*H₂O/ TSTPP*12HZ01 | 0.0 | 18.2 | 40.4 | 29.1 | 11.3 | 41.4 | 15200 |
| 11 | Ca(OAc)₂*H₂O/ CyDTA | 0.0 | 19,0 | 56.6 | 34.0 | 22.6 | 24.4 | 14900 |
| 12 | Ca(OAc)₂*H₂O/ BPhDS | 0.0 | 20.0 | 56.9 | 38.7 | 18.2 | 23.1 | 14600 |

**Table 2**

| Hydrogenation of sunflower oil methyl ester - composition in mol% - Continuation | | | | | | | | |
|---|---|---|---|---|---|---|---|---|
| **Ex** | **Catalyst precursor** | **C18:3** | **C18:2** | **C18:1** | ***Cis* C18:1** | ***Trans* C18:1** | **C18:0** | **TOF [h⁻¹]** |
| 13 | Ca(OAc)_{2/} BPyDCA | 0.0 | 20.2 | 57.4 | 36.9 | 20.5 | 22.4 | 14500 |
| 14 | Ca(OAc)₂* H₂O/ EDTANa₄*H₂O | 0.0 | 20.3 | 56.7 | 36.0 | 20.7 | 23.0 | 14400 |
| 15 | Ca(OAc)₂*H₂O/ BQC | 0.0 | 22.9 | 56.7 | 38.2 | 18.5 | 20.4 | 13500 |
| 16 | Ca(OAc)₂*H₂O/ BCDS | 0.0 | 23.2 | 55.6 | 38.9 | 16.7 | 21.2 | 13400 |
| 17 | Ca(OAc)₂*H₂O/ 2,6-PyDCAZ 2 | 0.0 | 26.2 | 56.4 | 44.3 | 12.1 | 17.4 | 12300 |
| 18 | Ca(OAc)₂*H₂O/ NTANₐ₃ ² | 0.0 | 34.3 | 50.0 | 41.1 | 8.9 | 15.7 | 9000 |

| | | | | | | | | |
|---|---|---|---|---|---|---|---|---|
| ¹0.88 mg (0.005 mmol) Ca(OAc)₂.H₂O; 6.19 mg (0.005 mmol) TSTPP.12H₂O (Ligand /Ca molar ratio = 1); 6.95 g (30 mmol of C=C units) of MESO; molar ratio of C=C units/Ca = 6 000; 10 ml of demineralized water; [Ca] = 20 ppm pH= 5.68; Addition of MESO to the aqueous catalyst solution results a two-phase system with a volume ratio of aqueous/organic phase = 10/7.7; stirring rate = 620 rpm. ² 0.88 mg (0.005 mmol) Ca(OAc)₂^{.}H₂O; 0.005 mmol of NTANa₃ or 2,6-PyDCA (Ligand /Ca molar ratio = 1); 6.95 g (30 mmol of C=_{C} units) of MESO; molar ratio of C=_{C} units/Ca = 6 000; 10 ml of demineralized water; [Ca] = 20 ppm; pH= 4.57 - 7.84; Addition of MESO to the aqueous catalyst solution results a two-phase system with a volume ratio of aqueous/organic phase = 10/7.7; stirring rate = 620 rpm. | | | | | | | | |

### Examples 19 to 25

### Effect of temperature on the Ca/EDTANa₄-and Ca/TSTPP-catalyzed hydrogenation

A commercially available sunflower oil methyl ester (MESO) was subjected to hydrogenation using various homogenous catalysts in aqueous/organic two phase systems under the following conditions:
- P_{H2} = 50 bar
- t = 10 min
- 1.47 mg (0.01 mmol) CaCl₂·2H₂O
- 3.98 mg (0.01 mmol) EDTANa₄·H₂O (EDTANa₄/Ca molar ratio = 1)
- ₀ 13.89 g (60 mmol of C=_{C} units) of MESO
- molar ratio of C=C units/Ca = 6 000
- 10 ml of demineralized water
- [Ca] = 40 ppm
- pH=4.12-5.71

Addition of MESO to the aqueous catalyst solution results a two-phase system with a volume ratio of aqueous/organic phase = 10/15.4; stirring rate = 620 rpm. The following Table 3 shows the effect of temperature on the Ca/EDTANa₄-and Ca/TSTPP-catalyzed hydrogenation of MESO in aqueous/organic two-phase systems

**Table 3**

| Hydrogenation of sunflower oil methyl ester-composition in mol% | | | | | | | | | |
|---|---|---|---|---|---|---|---|---|---|
| **Ex** | **Catalyst precursor** | **T** (°°C) | **C18:3** | **C18:2** | **C18:1** | ***Cis* C18:1** | ***Trans* C18:1** | **C18:0** | **TOF [h⁻¹]** |
| - | MESO | - | 1.3 | 59.0 | 36.5 | 36.5 | - | 3.2 | - |
| 19 | CaCl₂*2H₂O | 60 | 0.0 | 48.3 | 42.0 | 38.8 | 3.2 | 9.7 | 4300 |
| 20 | CaCl₂*2H₂O/ TSTPP*12H₂O¹ | 60 | 0.0 | 49.3 | 34.6 | 32.6 | 2.0 | 16.1 | 4650 |
| 21 | CaCl₂*H₂O/ EDTANa₄*2H₂O | 60 | 0.0 | 48.1 | 40.7 | 36.5 | 4.2 | 11.2 | 4400 |
| 22 | CaCl₂*H₂O/ EDTANa₄*H₂O | 80 | 0.0 | 38.9 | 46.3 | 38.6 | 7.7 | 14.8 | 7700 |
| 23 | CaCl₂* *H₂O/ EDTANa₄* H₂O | 100 | 0.0 | 28.4 | 53.4 | 39.2 | 14.2 | 18.2 | 11500 |
| 24 | CaCl₂*H₂O/ EDTANa₄* H₂O | 120 | 0.0 | 25.1 | 55.2 | 39.4 | 15.8 | 19.7 | 12700 |
| 25 | CaCl₂* H₂O/ EDTANa₄* H₂O | 130 | 0.0 | 13.6 | 59.6 | 39.1 | 20.5 | 26.8 | 16900 |

| | | | | | | | | | |
|---|---|---|---|---|---|---|---|---|---|
| ¹0.73 mg (0.005 mmol) CaCl₂^{.}2H₂O; 6.19 mg (0.005 mmol) TSTPP·12H₂O (TSTPP /Ca molar ratio = 1); 10 ml of demineralized water; [Ca] = 20 ppm; pH= 5.52. Addition of 6.95 g (30 mmol of C=C units) of MESO; molar ratio of C=_{C} units/Ca = 6 000; volume ratio of aqueous/organic phase = 10/7.7 | | | | | | | | | |

### Examples 26 to 29

### Effect of reaction time on the Ca/EDTA-catalyzed hydrogenation

A commercially available sunflower oil methyl ester (MESO) was subjected to hydrogenation using various homogenous catalysts in aqueous/organic two phase systems under the following conditions:
- T = 80 °c
- P_{H2} = 30 bar;
- 1.76 mg (0.01 mmol) Ca(OAC)₂·H₂O;
- 2.92 mg (0.01 mmol) EDTA (EDTA/Ca molar ratio = 1);
- 10 ml of demineralized water;
- [Ca] = 40 ppm;
- pH= 3.57 - 4.06;
- 6.95 g (30 mmol C=_{C} units) of MESO;
- molar ratio of C=C units/Ca= 3 000;
- volume ratio of the aqueous/organic phase = 10/7.7; stirring rate = 620 rpm.

The following Table 4 shows the effect of reaction time on the Ca/EDTA-catalyzed hydrogenation of MESO in aqueous/organic two-phase systems

**Table 4**

| Hydrogenation of sunflower oil methyl ester-composition in mol% | | | | | | | | | |
|---|---|---|---|---|---|---|---|---|---|
| **Ex** | **Catalyst precursor** | **t [min]** | **C18:3** | **C18:2** | **C18:1** | ***Cis* C18:1** | ***Trans* C18:1** | **C18:0** | **TOF [h -¹]** |
| - | MESO | | 1.3 | 59.0 | 36.5 | 36.5 | - | 3.2 | - |
| 26 | Ca(OAC)₂^{*} H₂O/ EDTA | 10 | 0.0 | 37.2 | 43.6 | 36.2 | 7.4 | 19.2 | 4200 |
| 27 | | 30 | 0.0 | 21.0 | 45.9 | 32.3 | 13.6 | 33.1 | 2400 |
| 28 | | 60 | 0.0 | 8.3 | 35.6 | 22.1 | 13.5 | 56.1 | 1600 |
| 29 | | 120 | 0.0 | 1.8 | 15.9 | 8.5 | 7.4 | 82.3 | 1200 |

### Examples 30 to 35

### Effect of C=C units/Ca molar ratio on the Ca/EDTA- and Ca/TSTPP-catalyzed hydrogenation

A commercially available sunflower oil methyl ester (MESO) was subjected to hydrogenation using various homogenous catalysts in aqueous/organic two phase systems under the following conditions:
- T=130°C
- ⁰ P_{H2} = 80 bar
- ₀ t = 5 min
- ₀ 1.76 mg (0.01 mmol) Ca(OAc)₂·H₂O
- ₀ 2.92 mg (0.01 mmol) EDTA (EDTA/Ca molar ratio = 1)
- ₀ 10 ml of demineralized water
- ₀ [Ca] = 40 ppm
- ₀ pH = 3.31-3.92

The following Table 5 shows the effect of C=C units/Ca molar ratio on the Ca/EDTA- and Ca/TSTPP-catalyzed hydrogenation of MESO in aqueous/organic two-phase systems.

**Table 5**

| Hydrogenation of sunflower oil methyl ester-composition in mol%; MR = molar ratio C=C/Ca¹ | | | | | | | | | |
|---|---|---|---|---|---|---|---|---|---|
| **Ex** | **Catalyst precursor** | **MR** | **C18:3** | **C18:2** | **C18:1** | ***Cis* C18:1** | ***Trans* C18:1** | **C18:0** | **TOF [h⁻¹]** |
| - | MESO | | 1.3 | 59.0 | 36.5 | 36.5 | - | 3.2 | - |
| 30 | Ca(OA_{C})₂^{*} H₂O/ TSTPP*12H₂O² | 20000 | 0.0 | 33.1 | 49.7 | 37.9 | 11.8 | 17.2 | 65400 |
| 31 | Ca(OAc)2^{*} H₂O/ TSTPP*12H2O 3 | 12000 | 0.0 | 19.8 | 45.7 | 31.1 | 14.6 | 34.5 | 58400 |

**Table 5**

| Hydrogenation of sunflower oil methyl ester-composition in mol%; MR = molar ratio C=C/Ca¹- Continuation | | | | | | | | | |
|---|---|---|---|---|---|---|---|---|---|
| **Ex** | **Catalyst precursor** | **MR** | **C18:3** | **C18:2** | **C18:1** | ***Cis* C18:1** | ***Trans* C18:1** | **C18:0** | **TOF h j** |
| 32 | Ca(OAc)₂^{*} H₂O/ EDTA | 20000 | 0.0 | 47.6 | 44.6 | 37.6 | 7.0 | 7.8 | 30600 |
| 33 | Ca(OAc)₂^{*} H₂O/ EDTA | 12000 | 0.0 | 34.4 | 49.1 | 38.0 | 11.1 | 16.5 | 37300 |
| 34 | Ca(OAc)₂^{*} H₂O/ EDTA | 6000 | 0.0 | 17.0 | 47.3 | 32.6 | 14.7 | 35.7 | 31200 |
| 35 | Ca(OAc)_{z}^{*} H_{z}0/ EDTA | 3000 | 0.0 | 14.9 | 30.8 | 22.3 | 8.5 | 54.3 | 18400 |

| | | | | | | | | | |
|---|---|---|---|---|---|---|---|---|---|
| ¹ Example 32: addition of 46.31 g (200 mmol C=C units) of MESO to the aqueous catalyst solution results a two-phase system with a volume ratio of the aqueous/organic phase of 10/51.5. Example 33: 27.78 g (120 mmol of C=_{C} units) of MESO; aqueous/organic phase = 10/30.9. Example 34: 13.89 g (60 mmol of C=_{C} units) of MESO; aqueous/organic phase = 10/15.4. Example 35: 6.95 g (30 mmol of C=C units) of MESO; aqueous/organic phase = 10/7.7. Stirring rate = 620 rpm. Z 0.88 mg (0.005 mmol) Ca(OAc)₂·H₂O; 6.19 mg (0.005 mmol) TSTPP·12H₂O (TSTPP /Ca molar ratio = 1); 10 ml of demineralized water; [Ca] = 20 ppm; pH= 5.57. Addition of 23.15 g (100 mmol of C=C units) of MESO to the aqueous catalyst solution results a two-phase system with a volume ratio of aqueous/organic phase = 10/25.7. ³0.88 mg (0.005 mmol) Ca(OAc)₂·H₂O; 6.19 mg (0.005 mmol) TSTPP·12H₂O; 10 ml of demineralized water; [Ca] = 20 ppm; pH= 5.38. Addition of 13.89 g (60 mmol of C=_{C} units) of MESO; volume ratio of aqueous/organic phase = 10/15.4. | | | | | | | | | |

### Examples 36 to 42

### Effect of molar ratio EDTA/Ca on the hydrogenation

A commercially available sunflower oil methyl ester (MESO) was subjected to hydrogenation using various homogenous catalysts in aqueous/organic two phase systems under the following conditions:
- ⁰ T = 80 °C;
- ⁰ P_{H2} = 30 bar;
- ₀ t= 10 min;
- ₀ 1.76 mg (0.01 mmol) Ca(OAc)₂·H₂O;
- 10 ml of demineralized water;
- [Ca] = 40 ppm; pH= 2.36 - 5.68;
- 6.95 g (30 mmol C=C units) of MESO; molar ratio of C=C units/Ca= 3 000; volume ratio of the aqueous/organic phase = 10/7.7;
- stirring rate = 620 rpm.

The following Table 6 shows the effect of molar ratio EDTA/Ca on the hydrogenation of MESO in aqueous/organic two-phase systems.

**Table 6**

| Hydrogenation of sunflower methyl ester - composition in mol%; MR = molar ratio EDTA/Ca | | | | | | | | | |
|---|---|---|---|---|---|---|---|---|---|
| **Ex** | **Catalyst precursor** | **MR** | **C18:3** | **C18:2** | **C18:1** | ***Cis* C18:1** | ***Trans* C18:1** | **C18:0** | **TOF [h⁻¹]** |
| - | MESO | | 1.3 | 59.0 | 36.5 | 36.5 | - | 3.2 | - |
| 36 | CaO(Ac)₂^{*} H₂O | 0 | 0.0 | 39.2 | 43.0 | 36.9 | 6.1 | 17.8 | 3800 |
| 37 | Ca(OAc)₂^{*} H₂O/ EDTA | 1 | 0.0 | 37.2 | 43.6 | 36.2 | 7.4 | 19.2 | 4200 |
| 38 | | 2 | 0.0 | 34.8 | 43.7 | 36.5 | 7.2 | 21.5 | 4600 |
| 39 | | 4 | 0.0 | 34.0 | 44.1 | 37.7 | 6.4 | 21.9 | 4750 |
| 40 | | 8 | 0.0 | 30.5 | 48.9 | 39.2 | 9.7 | 20.6 | 5400 |
| 41 | | 16 | 0.0 | 18.8 | 55.2 | 43.4 | 11.8 | 26.0 | 7500 |
| 42 | | 24 | 0.0 | 32.3 | 46.9 | 38.5 | 8.4 | 20.8 | 5100 |

### Examples 43 to 45

### Effect of pH value on the Ca/EDTA-catalyzed hydrogenation

A commercially available sunflower oil methyl ester (MESO) was subjected to hydrogenation using various homogenous catalysts in aqueous/organic two phase systems under the following conditions:
- ₀ T=80°C
- P_{H2} = 30 bar
- t= 10 min
- 1.76 mg (0.01 mmol) Ca(OAc)₂·H₂O
- 10 ml of demineralized water
- [Ca] = 40 ppm
- 6.95 g (30 mmol C=C units) of MESO
- molar ratio of C=C units/Ca= 3 000
- volume ratio of the aqueous/organic phase = 10/7.7
- stirring rate = 620 rpm

The following Table 7 shows the pH value on the Ca/EDTA-catalyzed hydrogenation of MESO in aqueous/organic two-phase systems.

**Table 7**

| Hydrogenation of sunflower oil methyl ester-composition in mol% | | | | | | | | | |
|---|---|---|---|---|---|---|---|---|---|
| **Ex** | **Catalyst precursor** | **pH** | **C18:3** | **C18:2** | **C18:1** | ***Cis* C18:1** | ***Trans* C18:1** | **C18:0** | **TOF [h -1]** |
| - | MESO | | 1.3 | 59.0 | 36.5 | 36.5 | - | 3.2 | - |
| 43 | Ca(OAc)₂^{*} H₂O/ EDTA | 2.36 | 0.0 | 18.8 | 55.2 | 43.4 | 11.8 | 26.0 | 7500 |
| 44 | | 7.0¹ | 0.0 | 19.8 | 52.5 | 41.6 | 10.9 | 27.7 | 7300 |
| 45 | | 10_{.91} | 0.0 | 32.5 | 46.9 | 38.6 | 8.3 | 20.6 | 5000 |

| | | | | | | | | | |
|---|---|---|---|---|---|---|---|---|---|
| ¹ Adjusted with 2% aqueous NaOH | | | | | | | | | |

### Examples 46 to 48

### Effect of H₂ partial pressure on the hydrogenation of MESO catalyzed by Ca/EDTA complexes

A commercially available sunflower oil methyl ester (MESO) was subjected to hydrogenation using various homogenous catalysts in aqueous/organic two phase systems under the following conditions:
- T = 80 °C;
- t= 10 min;
- 1.76 mg (0.01 mmol) Ca(OAc)₂^{.}H₂O;
- 46.75 mg (0.16 mmol) EDTA (EDTA/Ca molar ratio = 16);
- 10 ml of demineralized water;
- [Ca]=40 ppm;
- pH= 2.36 - 2.72;
- 6.95 g (30 mmol C=C units) of MESO;
- molar ratio of C=C units/Ca= 3 000;
- volume ratio of the aqueous/organic phase = 10/7.7;
- stirring rate = 620 rpm.

The following Table 8 shows the effect of hydrogen partial pressure on the hydrogenation of MESO catalyzed by Ca/EDTA complexes in aqueous/organic two-phase systems.

**Table 8**

| Hydrogenation of sunflower oil methyl ester - composition in mol% | | | | | | | | | |
|---|---|---|---|---|---|---|---|---|---|
| **Ex** | **Catalyst precursor** | **P_{H}2** | **C18:3** | **C18:2** | **C18:1** | ***Cis* C18:1** | ***Trans* C18:1** | **C18:0** | **TOF [h⁻¹]** |
| - | MESO | | 1.3 | 59.0 | 36.5 | 36.5 | - | 3.2 | - |
| 46 | Ca(OA_{C})₂^{*} H₂O/ EDTA | 30 | 0.0 | 18.8 | 55.2 | 43.4 | 11.8 | 26.0 | 7500 |
| 47 | | 50 | 0.0 | 17.6 | 42.5 | 33.1 | 9.4 | 39.9 | 7700 |
| 48 | | 80 | 0.0 | 17.3 | 35.5 | 27.6 | 7.9 | 47.2 | 7950 |

### Examples 49 to 50

### Recycling of Ca/EDTA catalysts

A commercially available sunflower oil methyl ester (MESO) was subjected to hydrogenation using various homogenous catalysts in aqueous/organic two phase systems under the following conditions:
- T = 130 °C;
- P_{H2}=80bar
- t= 5 min;
- 0.88 mg (0.005 mmol) Ca(OAc)₂·H₂O;
- 23.38 mg (0.08 mmol) EDTA (EDTA/Ca molar ratio = 16);
- 10 ml of demineralized water;
- [Ca] = 20 ppm;
- pH= 2.72;
- 11.58 g (50 mmol C=C units) of MESO;
- molar ratio of C=C units/Ca= 10 000;
- volume ratio of the aqueous/organic phase = 10/12.9;
- stirring rate = 620 rpm.

The aqueous catalyst layer (10 ml) of Example 49, after separation of the upper organic phase was re-used with addition of a new portion of 11.58 g (50 mmol C=C units) of MESO (Example 50) The results are shown in Table 9.

**Table 9**

| Hydrogenation of sunflower methyl ester-composition in mol%; molar ratio C=C/Ca = 10.000 | | | | | | | | |
|---|---|---|---|---|---|---|---|---|
| **Ex** | **Catalyst precursor** | **C18:3** | **C18:2** | **C18:1** | ***Cis* C18:1** | ***Trans* C18:1** | **C18:0** | **TOF [h -1]** |
| - | MESO | 1.3 | 59.0 | 36.5 | 36.5 | - | 3.2 | - |
| 49 | Ca(OAc)₂* H₂O/ EDTA | 0.0 | 7.8 | 37.3 | 23.0 | 14.3 | 54.9 | 63100 |
| 50 | Recycled catalyst of Example 49 | 0.0 | 7.7 | 37.3 | 23.7 | 13.6 | 55.0 | 63200 |

### Examples 51 to 55

### Effect of various main-group I-V metal catalyst precursors modified by EDTA on the hydrogenation of rapeseed oil methyl esters (MERO)

A commercially available rapeseed oil was transesterificated with methanol and the obtained rapeseed oil methyl ester (MERO) mixture was subjected to hydrogenation using various homogenous catalysts in aqueous/organic two phase systems under the following conditions:
- T = 120 °C;
- P_{H2} = 60 bar;
- t=l0min;
- 0.01 mmol of main-group metal catalyst precursor;
- 46.75 mg (0.16 mmol) EDTA (EDTA/Ca molar ratio = 16);
- 8.13 g (30 mmol C=C units) of MERO;
- molar ratio of C=C units/main group metal = 3 000;
- 15 ml of demineralized water;
- pH= 2.23 - 2.54;
- volume ratio of the aqueous/organic phase = 15/9.0;
- stirring rate = 620 rpm.

The following TablelO shows the effect of various main-group I-V metal catalysts precursors modified by EDTA on the hydrogenation of MERO in aqueous/organic two-phase systems.

**Table 10**

| Hydrogenation of rapeseed oil methyl ester (MERO) - composition in mol% | | | | | | | | |
|---|---|---|---|---|---|---|---|---|
| **Ex** | **Catalyst precursor** | **C18:3** | **C18:2** | **C18: 1** | ***Cis* C18:1** | ***Trans* C18:1** | **C18:0** | **TOF [h⁻¹]** |
| - | MERO | 7.8 | 18.6 | 72.0 | 71.4 | 0.6 | 1.6 | - |
| 51 | K(OAc)/EDTA | 4.0 | 12.6 | 67.4 | 54.7 | 12.7 | 16.0 | 2600 |
| 52 | Ca(OAc)₂·H₂O/ EDTA | 3.8 | 12.0 | 67.5 | 54.9 | 12.6 | 16.7 | 2700 |
| 53 | AlOH(OAc)₂·H₂O/ EDTA | 4.1 | 12.3 | 68.0 | 55.9 | 12.1 | 15.6 | 2500 |
| 54 | SnCl₂·2H₂O/ EDTA | 2.0 | 14.6 | 71.9 | 62.1 | 9.8 | 11.5 | 1800 |
| 55 | Bi(OAc)_{3/}EDTA | 4.6 | 13.4 | 69.0 | 58.5 | 10.5 | 13.0 | 3000 |

### Example 56 to 58

### Hydrogenation of rapeseed oil catalyzed by Ca/EDTA complexes

A commercially available rapeseed oil was subjected to hydrogenation using water-soluble Ca/EDTA catalytic complexes in aqueous/organic two phase systems under the following conditions:
- T = 130 °C;
- P_{H2} = 80 bar
- t= 3 h;
- 1.76 mg (0.01 mmol) Ca(OAc)₂·H₂O;
- 46.75 mg (0.16 mmol) EDTA;
- molar ration of EDTA/Ca = 16;
- 15 ml of demineralized water;
- [Ca] = 26 ppm;
- pH= 2.36 - 2.42;
- stirring rate = 820 rpm.

The following Table11 shows the hydrogenation of rapeseed oil catalyzed by water-soluble Ca/EDTA complexes in aqueous/organic two-phase systems. The composition of rapeseed oil before and after the hydrogenation reaction was determined after transesterification of the rapeseed oil triglycerides with methanol and analyzed the obtained rapeseed oil methyl esters mixture by gas chromatography.

**Table 11**

| Hydrogenation of rapeseed oil - composition in mol%; MR = molar ratio C=C/Ca¹ | | | | | | | | | |
|---|---|---|---|---|---|---|---|---|---|
| **Ex** | **Catalyst precursor** | **MR** | **C18:3** | **C18:2** | **C18:1** | ***Cis* C18:1** | ***Trans* C18:1** | **C18:0** | **TOF [h⁻¹]** |
| - | Rapeseed oil | - | 7.8 | 18.6 | 72.0 | 71.4 | 0.6 | 1.6 | - |
| 56 | Ca(OAC)₂·H₂O/ ED_{TA1} | 6000 | 3.0 | 11.0 | 64.5 | 50.0 | 14.5 | 21.5 | 400 |
| 57 | Ca(OAc)₂·H₂O/ EDTA² | 2000 | 2.5 | 5.4 | 47.2 | 24.9 | 22.3 | 44.9 | 290 |
| 58 | Ca(OAc)₂·H₂O/E D_{TA3} | 1000 | 0.0 | 0.0 | 6.5 | 3.3 | 3.2 | 93.5 | 300 |

| | | | | | | | | | |
|---|---|---|---|---|---|---|---|---|---|
| ¹ Example 56: 16.17 g (60 mmol C=C units) of rapeseed oil; volume ratio of the aqueous/organic phase = 15/17.8 ² Example 57: 5.39 g (20 mmol C=C units) of rapeseed oil; volume ratio of the aqueous/organic phase = 15/5.9 ³ Example 58: 140°C; P_{H2}= 90 bar; 20 ml of demineralized water; [Ca]= 20 ppm; 2.69 g (10 mmol C=C units) of rapeseed oil; volume ratio of the aqueous/organic phase = 20/2.9 | | | | | | | | | |

### Examples 59 to 62

### Hydrogenation of benzaldehyde using water-soluble Ca/fOTA and Ca/EDTANa₄·H₂O catalytic complexes

The following calcium-catalyzed benzaldehyde hydrogenation examples were carried out under various temperatures and in the presence of the water-soluble amine ligand modifiers EDTA and EDTANa ₄·H₂O in aqueous/organic two-phase systems. The reaction conditions were the following:
- P_{H2} = 50 bar;
- t=l0min;
- 1.76 mg (0.01 mmol) Ca(OAc)₂·H₂O
- 0.16 mmol EDTA or EDTANa ₄·H₂O ligand
- molar ratio of Ligand/Ca = 16
- 3.184 g (30 mmol) of benzaldehyde
- molar ratio of benzaldehyde/Ca = 3 000
- 15 ml of demineralized water;
- [Ca] = 27 ppm
- pH= 2.54 - 6.31
- volume ratio of aqueous/organic phase = 15/3.05;
- stirring rate = 620 rpm.

The following Table12 shows the effect of temperature and the water-soluble amine ligand modifiers on the hydrogenation of benzaldehyde in aqueous/organic two-phase systems. The turnover frequency (TOF) is defined as mole of converted benzaldehyde to give benzyl-alcohol (BA), toluene (T) and benzene (B) per mole of calcium per hour.

**Table 12**

| Hydrogenation of benzaldehyde using water-soluble Ca/EDTA and Ca/EDTANa₄·H₂O catalytic complexes | | | | | | | |
|---|---|---|---|---|---|---|---|
| **Ex** | **Catalyst precursor** | **T (°C)** | **Conv. (mol%)** | **Selectivity to BA (mol%)** | **Selectivity to T (mol%)** | **Selectivity to B (mol%)** | **TOF¹ [h -1]** |
| 59 | Ca(OAc)₂-H_{z}0 / EDTA | 80 | 28.2 | 69.3 | 29.3 | 1.4 | 5100 |
| 60 | Ca(OAc)₂·H₂O / EDTA | 100 | 47.3 | 54.8 | 43.2 | 2.0 | 8500 |
| 61 | Ca(OAC)₂·H₂O / EDTA | 120 | 73.8 | 32.3 | 66.0 | 1.7 | 13300 |
| 62 | Ca(OAc)₂·H₂O / EDTA Na₄·H₂O | 120 | 40.8 | 67.5 | 30.8 | 1.7 | 7300 |

### Examples 63 to 66

### Hydrogenation of benzene to cyclohexane (CHa) and cyclohexene (CHe) using water-soluble Ca/EDTA and Ca/EDTANa₄·H₂O catalytic complexes

The following calcium-catalyzed benzene hydrogenation examples were carried out under various temperatures and reaction times in the presence of the water-soluble amine ligand modifiers EDTA and EDTANa ₄·H₂O under various ligand/Ca molar ratios in aqueous/organic two-phase systems. The reaction conditions were the following:
- P_{H2} = 60 bar;
- 1.76 mg (0.01 mmol) Ca(OAC)₂·H₂O
- 2.343 g (30 mmol) of benzene;
- molar ratio of C=C units/Ca = 9000
- 20 ml of demineralized water;
- [Ca] = 20 ppm;
- pH=2.91 - 9.21;
- volume ratio of aqueous/organic phase = 20/2.67;
- stirring rate = 620 rpm.

The following Table13 shows the effect of temperature, reaction times in the presence of the water-soluble amine ligand modifiers EDTA and EDTANa ₄·H₂O under various ligand/Ca molar ratios in aqueous/organic two-phase systems. The turnover frequency (TOF) is defined as mole of hydrogenated C=_{C} units in benzene per mole of calcium per hour.

**Table 13**

| Hydrogenation of benzene using water-soluble Ca/EDTA and Ca/EDTANa₄·H₂O catalytic complexes; MR = molar ratio Ligand/Ca | | | | | | | | |
|---|---|---|---|---|---|---|---|---|
| **Ex** | **Catalyst precursor** | **T (°C)** | **t (min)** | **MR** | **Conv. (mol%)** | **Selectivity to Cha (mol%)** | **Selectivity to CHe (mol%)** | **TOF¹** [h**⁻¹]** |
| 63 | Ca(OAc)₂·H₂O/ EDTA | 100 | 20 | 16 | 26.3 | 96.5 | 3.5 | 7000 |
| 64 | Ca(OAc)₂·H₂O/ EDTANa₄·H₂O | 100 | 30 | 2 | 34.4 | 97.0 | 3.0 | 6100 |
| 65 | Ca(OAc)₂·H₂O/ EDTANa₄·H₂O | 120 | 30 | 2 | 46.8 | 95.2 | 4.8 | 8300 |
| 66 | Ca(OAc)₂·H₂O/ EDTANa₄·H₂O | 130 | 30 | 2 | 47.0 | 88.1 | 11.9 | 8100 |

## Claims

1. Process for the selective hydrogenation of (poly)unsaturated hydrocarbons into the respective saturated and/or monounsaturated species using homogenous water-soluble catalysts in a biphasic aqueous/organic solvent system, **characterised in that** said catalysts represent complexes of main-group I-V metals with water- soluble amine ligands.

2. Process according to Claim 1, **characterised in that** hydrocarbons are subjected to hydrogenation containing 6 to 54 carbon atoms and 2 to 5 double bonds.

3. Process according to Claim 1 and/or Claim 2, **characterised in that** hydrocarbons are subjected to hydrogenation selected from the group consisting of fatty acid C₁-C₂₂ alkyl- or polyol esters having 6 to 54 carbon atoms and 2 to 5 double bonds in the acyl moiety.

4. Process according to Claim 1 and/or Claim 2, **characterized in that** hydrocarbons are subjected to hydrogenation selected from the group consisting of methyl and/or ethyl and/or glycerol esters of linolenic acid, linoleic acid, eicosapentaenoic acid (EPA), docosahexaenoic acid (DHA) and their mixtures.

5. Process according to Claim 1 and/or Claim 2, **characterized in that** hydrocarbons are subjected to hydrogenation selected from the group consisting of aliphatic-, aromatic- arylaliphatic, unsaturated aliphatic or α,β-unsaturated aldehydes

6. Process according to Claim 1 and/or Claim 2, **characterised in that** hydrocarbons are subjected to hydrogenation selected from the group of aromatic compounds at least one of the following general formulas 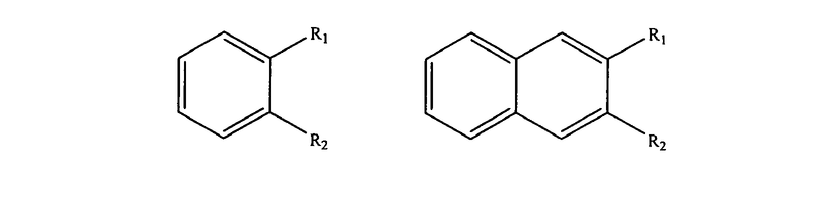 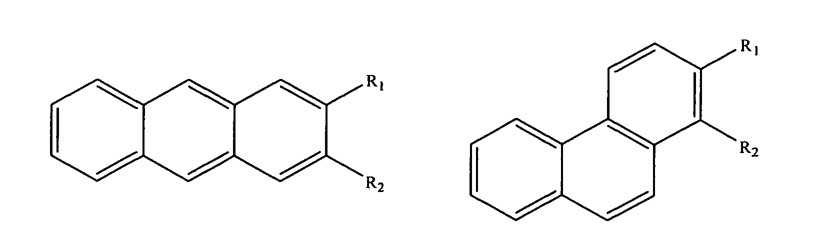 in which R¹ and R^{Z} independently from each other represent hydrogen or an alkyl, ester, alkoxy, aldehyde, keto, acid, amide, nitril, amino, nitro, halide, and/or hydroxy group, or their geometric isomers.

7. Process according to any of the preceding Claims 1 to 6, **characterised in that** homogenous catalysts derived from main-group II are employed.

8. Process according to any of the preceding Claims 1 to 7, **characterised in that** homogenous calcium catalysts are employed.

9. Process according to any of the preceding Claims 1 to 8, **characterised in that** homogenous main group II catalysts are employed, comprising at least one water-soluble amine ligand selected from the group consisting of ethylenediaminetetraacetic acid (EDTA), ethylenediaminetetraacetic acid tetrasodium salt (EDTANa₄), meso-tetra-(sulfonatophenyl)porphine tetrasodium salt (TSTPP), trans-1,2-diaminocyclohexane-N,N,N",N'-tetraacetic acid (CyDTA), 2,2'-biquinoline-4,4'dicarboxylic acid dipotassium salt (BQC), 2,2-bipyridine-4,4-dicarboxylic acid (BPyDCA), bathophenanthrolinedisulfonic acid disodium salt (BPhDS), bathocuproinedisulfonic acid disodium salt (BCDS), diethylenetriaminepentaacetic acid pentasodium salt (DTPANa₅), nitrilotriacetic acid trisodium salt (NTANa₃) and 2,6-pyridinedicarboxylic acid (2,6-PyDCA).

10. Process according to any of the preceding Claims 1 to 9, **characterised in that** the hydrogenation is conducted in the presence of co-catalysts selected from the group consisting of cationic, zwitterionic (amphoteric), nonionic and anionic surfactants, ionic liquids and phase transfer catalysts.

11. Complexes of main-group I-V metals and water-soluble amine ligands.

12. Complexes according to Claim 11, **characterised in that** the water-soluble ligands are selected from the group consisting of ethylenediaminetetraacetic acid (EDTA), ethylenediaminetetraacetic acid tetrasodium salt (EDTANa₄), meso-tetra(sulfonatophenyl)-porphine tetrasodium salt (TSTPP), trans-1,2-diaminocydohexane-N,N,N',N'-tetraacetic acid (CyDTA), 2,2'-biquinoline-4,4'di-carboxylic acid dipotassium salt (BQC), 2,2-bipyridine-4,4-dicarboxylic acid (BPyDCA), bathophenanthrolinedisulfonic acid disodium salt (BPhDS), bathocuproinedisulfonic acid disodium salt (BCDS), diethylenetriaminepentaacetic acid pentasodium salt (DTPANa₅), nitrilotriacetic acid trisodium salt (NTANa₃) and 2,6-pyridinedicarboxylic acid (2,6-PyDCA).

13. Use of said complexes according to Claim 11 as homogenous catalysts for the hydrogenation of (poly)unsaturated hydrocarbons, aldehydes and aromatic compounds.

14. Use according to Claim 13, **characterised in that** polyunsaturated fatty acid esters are transformed into saturated and/or monounsaturated fatty acid esters, of aldehydes into respective alcohols and of aromatic compounds into respective saturated cyclic compounds.
